# EUROPÄISCHE PATENTSCHRIFT

(11) **EP 2 106 266 B1**
(45) Veröffentlichungstag und Bekanntmachung des Hinweises auf die Patenterteilung: **24.09.2014**
(21) Anmeldenummer: 07856948.0
(22) Anmeldetag: 19.12.2007
(51) Int. Cl.: A61M 1/16, A61M 1/36

(54) **Verfahren zum Primen eines Blutschlauchsatzes**
Method for priming a blood line set
Procédé d'amorçage d'un ensemble de tubes sanguins

(30) Priorität: 22.12.2006 DE 102006061184
(43) Veröffentlichungstag der Anmeldung: 07.10.2009
(73) Patentinhaber: Fresenius Medical Care Deutschland GmbH, 61352 Bad Homburg (DE)
(72) Erfinder: BIESEL, Wolfgang, 66564 Ottweiler (DE); MILLAN GALANTE, Maria, 61352 Bad Homburg (DE)
(74) Vertreter: Laufhütte, Dieter
(86) Internationale Anmeldenummer: PCT/EP2007/011225
(87) Internationale Veröffentlichungsnummer: WO 2008/077573

(56) Entgegenhaltungen:
- EP-A- 0 069 246
- WO-A-03/006139
- DE-A1- 10 151 343
- DE-C1- 10 011 208
- DE-T2- 69 634 724
- DE-U1- 8 525 525
- FR-A- 2 593 068
- GB-A- 2 003 449
- US-A- 4 955 508

## Beschreibung

Die vorliegende Erfindung betrifft ein Verfahren zum Primen eines Blutschlauchsatzes, welcher eine venöse und eine arterielle Leitung umfasst.

Solche Blutschlauchsätze kommen bei extrakorporalen Bluttherapien wie z. B. der Hämodialyse zum Einsatz und bilden dabei den extrakorporalen Blutkreislauf. Als arterielle und venöse Leitung eines solchen Blutschlauchsatzes werden üblicherweise steril verpackte Einmalartikel verwendet, welche vor der Behandlung gefüllt und gespült werden müssen. Dieses Priming genannte Füllen und Spülen des Blutschlauchsatzes dient der Vermeidung eines Kontaktes des Blutes mit Luft bei der extrakorporalen Therapie sowie der Reinigung des Blutschlauchsatzes sowie des Dialysators bzw. des Dialysefilters. Hierzu werden die arterielle und die venöse Leitung, nachdem sie filterseitig mit dem Dialysator verbunden wurden, mit einer Primingflüssigkeit, d.h. einer physiologischen Lösung, gefüllt und so entlüftet. Nach dem Füllen werden die patientenseitigen Enden der venösen und arteriellen Leitungen üblicherweise direkt oder über ein Verbindungselement miteinander verbunden, so dass die Primingflüssigkeit in diesem Kreislauf aus arterieller Leitung, Dialysator und venöser Leitung zirkulieren kann, bis der Patient an das System angeschlossen wird. Nach dem Anschluß des Patienten wird die Primingflüssigkeit durch das einströmende Blut verdrängt, so dass ein möglicher Kontakt des Blutes mit der Luft auf ein Minimum reduziert wird. Hierbei ist das in dem Blutschlauchsatz zirkulierende Volumen jedoch durch das Volumen des Blutschlauchsatzes fest vorgegeben, so dass es je nach Sterilisationsverfahren und/oder verwendetem Filter bzw. Füllstoffen des Filters aus der Produktion zu klein sein kann, um eine ausreichende Reinigungs- und Entlüftungswirkung zu erzielen. Zudem ist das Umkonnektieren der Anschlüsse sowie das Anbringen bzw. Öffnen und Schließen der Ventile anfällig für Bedienungsfehler der Dialyseschwester.

Aus U.S. 4,955,508 ist weiterhin ein Primingverfahren bekannt, bei welchem ein Beutel mit Primingflüssigkeit mit zumindest zwei Kammern zum Einsatz kommt. Eine erste Kammern ist dabei mit Flüssigkeit gefüllt und verfügt über zwei separate Eingänge, welche unten am Beutel angeordnet sind. Die zweite Kammer ist leer und verfügt nur über einen Eingang. Zum Füllen des Blutschlauchsatzes wird nun die arterielle Leitung des Blutschlauchsatzes mit einem der Eingänge der vollen Kammer verbunden, während die venöse Leitung mit dem Eingang der leeren Kammer verbunden wird. Die Flüssigkeit aus der vollen Kammer fließt so in den Blutschlauchsatz und von dort in die leere Kammer gepumpt, so dass der Blutschlauchsatz befüllt und gespült wird. Daraufhin wird die venöse Leitung von dem Eingang der zweiten Kammer gelöst und mit dem zweiten Eingang der ersten Kammer verbunden, woraufhin die Primingflüssigkeit durch die erste Kammer zirkuliert. Soll die gebrauchte Primingflüssigkeit vor dem Anschluß an den Patienten durch frische ersetzt werden, muss wiederum eine der Leitungen abgenommen und an den Eingang einer dritten Kammer angeschlossen werden. So ergibt sich zwar ein großes Flüssigkeitsvolumen, welches im Blutschlauchsatz zirkulieren kann und damit die Reinigungswirkung des Verfahrens erhöht. Dieses ist aber durch die im Beutel vorhandene Menge im wesentlichen vorgegeben, so dass es nicht an die eigentlich benötigte Menge angepaßt werden kann. Gleichzeitig ist aber eine Vielzahl an Konnektionsvorgängen nötig, welche zu Bedienungsfehlern führen können und zudem immer die Gefahr einer Kontamination des Blutschlauchsatzes beinhalten. Zudem kann es aufgrund der Vielzahl von unterschiedlichen Anschlüssen an dem Beutel zu Verwechslungen kommen. Auch ist der Transport und andere Vorgänge wie z. B. die Sterilisation der Beutel kompliziert, da diese mit Flüssigkeit gefüllt sind.

Aus der WO 03/006 139 A1 ist es weiterhin bekannt, die arterielle und die venöse Leitung über eine Konnektoranordnung mit einem Beutel mit Primingflüssigkeit zu verbinden. Der Blutschlauchsatz wird befüllt, indem durch Betreiben der Blutpumpe Primingflüssigkeit über die eine Leitung aus dem Beutel abgezogen wird, während die im Blutschlauchsatz vorhandene Luft über die andere Leitung in den Beutel geschoben wird. Nach dem Befüllen wird die Primingflüssigkeit dann in dem Kreislauf aus den Leitungen, dem Dialysator und dem Beutel zirkuliert. Als alternative Quelle für die Primingflüssigkeit wird dabei eine über eine Zuleitung mit der venösen Leitung in Verbindung stehende Substituat-Quelle erwähnt.

Aufgabe der vorliegenden Erfindung ist es daher, ein Verfahren zum Primen eines Blutschlauchsatzes zur Verfügung zu stellen, welches einfacher, sicherer und ökonomischer durchzuführen ist. Ebenso ist es Aufgabe der vorliegenden Erfindung, einen entsprechenden Blutschlauchsatz bzw. Beutel zur Verfügung zu stellen.

Erfindungsgemäß wird diese Aufgabe von einem Verfahren gemäß Anspruch 1 sowie einem Blutschlauchsatz gemäß Anspruch 14 gelöst.

Dabei umfasst der Blutschlauchsatz eine venöse und ein arterielle Leitung, deren patientenseitige Anschlüsse mit zwei separaten Eingängen einer Kammer insbesondere eines Beutels in Verbindung stehen und deren filterseitige Anschlüsse mit einem Dialysator in Verbindung stehen, wobei das erfindungsgemäße Verfahren die Schritte umfasst: Befüllen der venösen Leitung und der arteriellen Leitung über eine Zuleitung, wobei Primingflüssigkeit über die Leitungen durch die Eingänge in die Kammer fließt, wobei die Kammer (40) zu Beginn der Primings leer ist, sowie Zirkulieren der Primingflüssigkeit in dem Kreislauf aus den Leitungen, dem Dialysator und der Kammer über eine Pumpe, so dass einer der Eingänge der Kammer als Eingang und der andere als Ausgang fungiert.

Durch das erfindungsgemäße Verfahren muss zwischen dem Befüllen des Blutschlauchsatzes und dem Zirkulieren nicht mehr umkonnektiert werden, so dass sich das Verfahren für das Behandlungspersonal vereinfacht, Konnektierfehler vermieden werden und die damit verbundenen Kontaminationsgefahren entfallen. Die Primingflüssigkeit wird hierfür über eine Zuleitung so in den Blutschlauchsatz eingeleitet, dass sie in die venöse und arterielle Leitung fließt bzw. gepumpt wird. Die Zuleitung ist hierfür mit einem Zuleitungspunkt entweder in der arteriellen oder der venösen Leitung verbunden, an dem der Flüssigkeitsstrom aus der Zuleitung in beide Richtungen durch die Leitungen in die Kammer fließen kann. Sobald soviel Flüssigkeit in die Kammer geflossen ist, dass die beiden separaten Eingänge durch die in der Kammer angesammelte Flüssigkeit miteinander verbunden sind, kann mit dem Zirkulieren begonnen werden. Hierzu sind die beiden separaten Eingänge vorteilhafterweise an der Unterseite der Kammer angeordnet. Z. B. kann dabei ein Beutel verwendet werden, an dessen Unterseite die Eingänge angeordnet sind, während an seiner Oberseite ein Aufhängelement zum Aufhängen des Beutels angeordnet ist.

Im Vergleich zu Primingverfahren, bei welchen die patientenseitigen Anschlüsse der venösen und der arteriellen Leitung zum Zirkulieren der Primingflüssigkeit entweder direkt oder über einen Adapter miteinander verbunden werden, hat das Befüllen über die Zuleitung und das Zirkulieren über die Kammer zudem den Vorteil, dass - wenn nötig - ein erheblich größeres Volumen an Primingflüssigkeit zirkuliert werden kann. Je nach Sterilisationsverfahren und/oder verwendetem Filter bzw. Füllstoffen des Filters aus der Produktion kann so das Volumen der Primingflüssigkeit groß oder klein gehalten werden und damit an die benötigte Reinigungswirkung angepaßt werden. Hierdurch ergibt sich eine erheblich bessere Spülung und Reinigung sowohl des Blutschlauchsatzes als auch des Dialysators, sowie eine verbesserte Ausspülung eventueller Verunreinigungen. Auch ergibt sich eine verbesserte Entlüftung, da über die Kammer ein Zirkulieren mit großen Flussraten möglich ist, so dass Mikrobläschen besser mobilisiert und getrennt werden. Ebenso werden hierdurch Fremdstoffe und Teilchen aus dem Blutschlauchsatz und dem Dialysator besser entfernt, welche sich dann in der Kammer durch Sedimentation ablagern. Hierzu reichen die Eingänge der Kammer vorteilhafterweise schlauchartig in diese hinein, so dass sich Fremdstoffe noch besser absetzen können. Zudem ist auch die Rezirkulation ohne großen Aufwand möglich, da insbesondere auf Adapter für diesen Zweck verzichtet werden kann. Damit ergibt sich ein äußerst einfaches Verfahren, bei dem Bedienfehler so gut wie ausgeschlossen sind.

Vorteilhafterweise wird nach dem Zirkulieren die gebrauchte Primingflüssigkeit im Blutschlauchsatz durch Nachfüllen frischer Primingflüssigkeit aus der Zuleitung in die venöse und in die arterielle Leitung ersetzt, wobei die gebrauchte Primingflüssigkeit durch die Eingänge in die Kammer abfließt. Dieser Nachfüllschritt ist mit dem ersten Befüllschritt im wesentlichen identisch. So kann vor dem Anschließen des Blutschlauchsatzes an den Patienten dafür gesorgt werden, dass die während des Spül- und Zirkuliervorgangs möglicherweise verunreinigte Primingflüssigkeit im Blutschlauchsatz durch frische Primingflüssigkeit ersetzt wird. Auch hierzu ist wiederum kein Umkonnektieren der Anschlüsse nötig. Auch ist kein zusätzlicher Behälter zur Aufnahme der gebrauchten Primingflüssigkeit nötig. So ergibt sich wiederum ein einfaches und sicheres Verfahren.

Vorteilhafterweise wird beim Befüllen oder Nachfüllen des erfindungsgemäßen Verfahrens die Primingflüssigkeit über die Zuleitung stromabwärts einer Pumpe in einer der Leitungen zugegeben. Der Flüssigkeitsstrom aus der Zuleitung kann so von diesem Zuleitungspunkt aus in eine Richtung durch den Druck aus der Zuleitung in Richtung Kammer fließen, während er in die andere Richtung durch die Pumpe zum anderen Eingang der Kammer gepumpt wird. Je nach Anordnung des Zuleitungspunkts und Laufrichtung der Pumpe wird so die Primingflüssigkeit entweder direkt in die Kammer oder zuerst durch den Dialysator gepumpt. Vorteilhafterweise befindet sich dabei der Zuleitungspunkt in der arteriellen Leitung vor der Pumpe, so dass diese vorwärts laufen kann und die Flüssigkeit aus der Zuleitung über den Dialysator in die Kammer pumpt, während der Flüssigkeitsstrom in die andere Richtung direkt in die Kammer fließt. Alternativ kann die Flüssigkeit aber auch in der arteriellen Leitung zwischen Pumpe und Dialysator zugegeben werden, wobei die Pumpe dann jedoch rückwärts laufen muss.

Weiterhin vorteilhafterweise können bei dem erfindungsgemäßen Verfahren die beiden Eingänge der Kammer jeweils über Ventile geöffnet und geschlossen werden, wobei vorteilhafterweise Klemmen zum Einsatz kommen. Dabei werden die Eingänge vorteilhafterweise geschlossen, bevor die venöse und arterielle Leitung dekonnektiert werden, um ein Ausfließen der in der Kammer gespeicherten Flüssigkeit zu vermeiden. Zudem können die Klemmen beim Befüllen bzw. Nachfüllen die Flüssigkeitsströme durch den Blutschlauchsatz regeln.

Vorteilhafterweise wird beim erfindungsgemäßen Befüllen oder Nachfüllen zunächst bei ausgeschalteter Pumpe der Abschnitt des Blutschlauchsatzes ohne Pumpe, insbesondere die arterielle Leitung, befüllt, und daraufhin der Abschnitt mit Pumpe, insbesondere die venöse Leitung, durch Betreiben der Pumpe. So ergibt sich ein sehr einfaches Verfahren, bei der z.B. die arterielle Leitung zuerst befüllt wird, indem Primingflüssigkeit aus der Zuleitung direkt vom Zuleitungspunkt über die arterielle Leitung in den Beutel läuft. Ist die arterielle Leitung gefüllt, kann durch Einschalten der Pumpe auch die venöse Leitung mit Primingflüssigkeit aus der Zuleitung gefüllt werden, welche über den Dialysator und die venöse Leitung in den Beutel fließt. Dieses Verfahren ist insbesondere von Vorteil, wenn die Primingflüssigkeit mittels Schwerkraft aus einem Beutel zugeführt wird, da die Fließraten hierzu nicht kontrolliert werden müssen.

Dabei wird vorteilhafterweise ein an dem Eingang, welcher mit dem patientenseitigen Anschluß der arteriellen Leitung verbunden ist, angeordnetes Ventil geschlossen, um die venöse Leitung über die Pumpe zu befüllen. So läuft man nicht Gefahr, während des Befüllens der venösen Leitung über die Pumpe Primingflüssigkeit wieder aus der arteriellen Leitung abzusaugen. Damit muss zum Befüllen des Blutschlauchsatzes lediglich ein einziges Ventil geschlossen werden, welches dann zum Zirkulieren wieder geöffnet wird. Weitere Schritte des Öffnens und Schließens von Ventilen sind aber nicht nötig, so dass sich ein sehr einfaches und sicheres Primingverfahren ergibt.

Alternativ kann sowohl die arterielle Leitung als auch die venöse Leitung parallel befüllt werden, indem Primingflüssigkeit aus der Zuleitung gleichzeitig in der Abschnitt ohne Pumpe läuft und von der Pumpe in den Abschnitt mit Pumpe gepumpt wird. Hierbei muss natürlich die von der Pumpe geförderte Flüssigkeitsmenge kleiner sein als die Flüssigkeitsmenge, welche aus der Zuleitung zufließt. Damit ist das Befüllen des Blutschlauchsatzes in nur einem Bedienschritt möglich, ohne dass hierzu an venöser und arterieller Leitung Veränderungen vorgenommen werden müssten. Damit werden Bedienfehler effektiv verhindert. Auch ist so das Umschalten zwischen Befüllen und Zirkulieren in nur einem Bedienschritt möglich, indem der Zustrom aus der Zuleitung abgeschaltet wird. Auch dies erhöht die Sicherheit und Effizienz des Verfahrens.

Vorteilhafterweise kann bei dem erfindungsgemäßen Verfahren die Zuleitung über ein Ventil geöffnet und geschlossen werden. Als Ventil kann dabei vorteilhafterweise eine Klemme dienen, welche an der Zuleitung angebracht ist. Hierdurch kann der Flüssigkeitsstrom aus der Zuleitung entsprechend den Bedürfnissen beim Befüllen und Zirkulieren eingestellt werden. Der nötige Druck für das Zuleiten der Primingflüssigkeit aus der Zuleitung entsteht dabei entweder durch die Schwerkraft oder aber durch eine in der Zuleitung angeordnete Zuleitungspumpe. Für das Befüllen mittels Schwerkraft eignet sich insbesondere die erste Befüllmethode, bei welcher zuerst z.B. die arterielle Leitung bei abgeschalteter Blutpumpe befüllt wird, und die venöse Leitung erst danach über die Blutpumpe befüllt wird.

Vorteilhafterweise wird in dem erfindungsgemäßen Verfahren die Primingflüssigkeit über eine Pumpe durch die Zuleitung gepumpt. So läßt sich der durch die Zuleitung zufließende Flüssigkeitsstrom genau regeln, so dass insbesondere das parallele Befüllen des Blutschlauchsatzes durch die entsprechende Einstellung der Pumpraten der in der Zuleitung und dem extrakorporalen Blutkreislauf angeordneten Pumpen einfach durchzuführen ist. Dabei wird über die Zuleitung eine größere Menge an Primingflüssigkeit zugeführt, als z.B. durch die venöse Leitung gepumpt wird, so dass die restliche Flüssigkeit direkt über die arterielle Leitung zum Beutel fließt. Die Pumpe kann dann auch das Ventil in der Zuleitung ersetzen, da über die üblicherweise verwendeten Rollenpumpen die Zuleitung ebenfalls abgesperrt werden kann.

Neben dem Zuleiten von Primingflüssigkeit während des Befüllens oder Nachfüllens des Blutschlauchsatzes ist es auch während des Zirkulierens der Flüssigkeit möglich, eine gewisse Menge an Primingflüssigkeit durch Öffnen des Ventils in der Zuleitung bzw. Verwenden der Pumpe zuzugeben, z. B. um im Dialysator austretende Flüssigkeit zu ersetzen. Durch diese Konvektion werden filtergängige Verunreinigungen über die Membran des Filters in das Dialysat ausgeschieden. Hierdurch wird der Filter effektiv gespült.

Vorteilhafterweise steht die Zuleitung mit einer zweiten Kammer insbesondere eines zweiten Beutels mit Primingflüssigkeit in Verbindung. So kann die Primingflüssigkeit auf einfache Weise zugegeben werden, wo insbesondere beim Befüllen über die Schwerkraft der zweite Beutel in einer erhöhten Position angeordnet wird, so dass die Primingflüssigkeit in den Blutschlauchsatz fließt.

Alternativ kann die in die Zuleitung geleitete Primingflüssigkeit auch on-line aus dem Dialysat gewonnen werden. Hierdurch ist keine zusätzliche Quelle für die Primingflüssigkeit nötig, was das Verfahren vereinfacht und Kosten spart, da das Dialysat und die entsprechende Apparatur ohnehin vorhanden sind. Hier kommt dann vorteilhafterweise eine Pumpe zum Einsatz, welche die Primingflüssigkeit durch die Zuleitung pumpt.

Vorteilhafterweise steht die Zuleitung dabei mit einem Substituat-Port in Verbindung. Dieser ist üblicherweise bereits mit einer Pumpe versehen, so dass ein genaues Ansteuern der durch die Zuleitung zufließenden Primingflüssigkeit ohne zusätzlichen Aufwand möglich ist. Der Substituat-Port stellt dabei physiologische Flüssigkeit zur Verfügung. Diese physiologische Flüssigkeit kann aber ebenso zum Primen verwendet werden, so dass hier apparativer Aufwand gespart werden kann.

Vorteilhafterweise werden bei dem erfindungsgemäßen Verfahren zur Blutbehandlung nach dem Primen zuerst der patientenseitige Anschluß der arteriellen Leitung von dem Eingang der Kammer getrennt und mit dem Patienten verbunden, dann die im Blutschlauchsatz verbliebene Primingflüssigkeit in die Kammer gepumpt, wobei Blut in den Blutschlauchsatz einfließt, und daraufhin auch der patientenseitige Anschluß der venösen Leitung mit dem Patienten verbunden. So wird die venöse Leitung erst dann mit dem Patienten verbunden, wenn der extrakorporale Kreislauf komplett mit Blut gefüllt ist. Auch hierzu sind nur eine minimale Anzahl an Konnektiervorgängen nötig, so dass das Verfahren einfach und sicher durchgeführt werden kann. Auch ist kein weiterer Behälter notwendig, in den die Primingflüssigkeit abgelassen werden müßte.

Alternativ können nach dem Nachfüllen frischer Primingflüssigkeit zur Blutbehandlung die patientenseitigen Anschlüsse der arteriellen und der venösen Leitung von den Eingängen der Kammer getrennt und mit dem Patienten verbunden werden, wobei im Blutschlauchsatz verbliebene Primingflüssigkeit zumindest teilweise dem Patienten zugeführt wird. Durch dieses im wesentlichen gleichzeitige Konnektieren beider Anschlüsse wird saubere Primingflüssigkeit in der Menge, in der Blut entnommen wird, dem Patienten zugegeben. Dies ist insbesondere bei kreislaufinstabilen Patienten von Vorteil, da sie keinen Flüssigkeitsverlust erleiden.

Verfahren nach einem der vorangegangenen Ansprüche, wobei dem Patienten während der Blutbehandlung Primingflüssigkeit über die Zuleitung z.B. als Bolusinfusion verabreicht wird. Dadurch, dass sowieso eine Zuleitung für Primingflüssigkeit zum Blutschlauchsatz vorhanden ist, verfügt das Behandlungspersonal während der Blutbehandlung jederzeit über die Möglichkeit, dem Patienten z.B. bei Kreislaufproblemen Flüssigkeit zuzuführen, ohne dass hierfür z.B. ein neuer Beutel mit physiologischer Lösung konnektiert werden müsste. Hier muss lediglich das entsprechende Ventil in der Zuleitung geöffnet und wieder geschlossen werden bzw. die entsprechende Pumpe betätigt werden.

Vorteilhafterweise wird bei dem erfindungsgemäßen Verfahren Blut, welches nach Abnehmen des patientenseitigen Anschlußes der arteriellen Leitung vom Patienten in dem Blutschlauchsatz verblieben ist, insbesondere durch Zugeben von Primingflüssigkeit aus der Zuleitung dem Patienten über die venöse Leitung wieder zugeführt. So kann das gesamte dem Patienten entnommene Blut auch wieder in den Patienten zurückgeführt werden. Zudem ist es so möglich, dem Patienten Flüssigkeit wieder zuzuführen. Hierzu kann z.B. während der Ultrafiltration im Dialysator entnommene Flüssigkeit zumindest teilweise durch Primingflüssigkeit aus der Zuleitung ersetzt werden, ohne dass hierzu umkonnektiert werden müsste. Hierzu wird, nachdem das Blut dem Patienten zurückgeführt wurde, solange Primingflüssigkeit nachgefördert, wie notwendig ist, um dem Patienten die entsprechende Menge an physiologischer Lösung zuzuführen.

Bei dem erfindungsgemäßen Verfahren ist die Kammer zu Beginn des Primings leer. Insbesondere wenn ein Beutel verwendet wird, hat dies den großen Vorteil, dass der Beutel mit der Kammer zur Aufnahme der Primingflüssigkeit anfangs leer ist und dadurch erheblich leichter transportiert und sterilisiert werden kann.

Weiterhin vorteilhafterweise kommen dabei ein Beutel und ein Blutschlauchsatz zum Einsatz, welche zumindest teilweise vorkonnektiert und in ihrer Gesamtheit sterilisiert sind. So wird eine unnötige Gefährdung des Patienten durch spätere Konnektionsvorgänge vermieden.

Weiterhin vorteilhafterweise wird bei dem erfindungsgemäßen Verfahren ein Beutel mit nur einer Kammer verwendet, der dann vorteilhafterweise an seiner Unterseite die zwei separaten Eingänge und an seiner Oberseite ein Aufhängeelement umfasst. So besteht bezüglich der Anschlüsse keine Verwechslungsgefahr mehr, da nur zwei Anschlüsse vorhanden sind, welche identisch verwendet werden können. Die zum Primen benötigte physiologische Flüssigkeit kommt dabei vorteilhafterweise aus einem anderen Beutel.

Weiterhin vorteilhafterweise umfasst die vorliegende Erfindung einen Blutschlauchsatz mit einem Beutel mit einer Kammer mit zwei separaten Eingängen sowie mit einer venösen Leitung und einer arteriellen Leitung, welche mit den zwei separaten Eingängen lösbar verbindbar sind, wobei an einer der Leitungen eine Zuleitung angeordnet ist. Hierdurch kann mit dem erfindungsgemäßen Blutschlauchsatz das erfindungsgemäße Verfahren durchgeführt werden, bei welchem die Primingflüssigkeit über die Zuleitung zugegeben wird und dann über die arterielle und die venöse Leitung in den Beutel läuft. Vorteilhafterweise ist die Zuleitung dabei fest mit der arteriellen oder der venösen Leitung verbunden, da die Zuleitung nicht vom Blutschlauchsatz getrennt werden muss. Vorteilhafterweise ist die Zuleitung dabei an einem Zuleitungspunkt insbesondere über einen T-Konnektor in die arterielle oder die venöse Leitung integriert. Alternativ kann die Zuleitung aber auch über eine lösbare Verbindung mit der Leitung verbindbar oder verbunden sein. Weiterhin vorteilhafterweise ist die Zuleitung dabei in der arteriellen Leitung vor einem Pumpensegment angeordnet. Hierdurch läßt sich wiederum mit diesem erfindungsgemäßen Blutschlauchsatz das erfindungsgemäße Verfahren besonders vorteilhaft durchführen.

Die vorliegende Erfindung umfasst weiterhin einen Blutschlauchsatz mit einem Beutel mit einer Kammer mit zwei separaten Eingängen sowie mit einer venösen Leitung und einer arteriellen Leitung, welche mit den zwei separaten Eingängen lösbar verbindbar sind. Dabei ist zumindest eine der Leitungen erfindungsgemäß mit dem leeren Beutel vorkonnektiert und mit diesem gemeinsam steril verpackt. Hierdurch können unnötige Gefährdungen des Patienten durch spätere Konnektionsvoränge des Beutels mit dieser Leitung vermieden werden. Die Verwendung eines solchen Beutels mit zumindest einer vorkonnektierten Leitung wird durch das erfindungsgemäße Verfahren möglich, da hierbei der Beutel zu Beginn des Primens leer ist und so gemeinsam mit der Leitung sterilisiert und verpackt werden kann und zum Befüllen und Zirkulieren der Primingflüssigkeit auch nicht mehr umkonnektiert werden muss. Um das Einlegen des Schlauchsets in die Dialysemaschine zu erleichtern, ist dabei vorteilhafterweise nur eine der Leitungen mit dem Beutel vorkonnektiert, wobei aber beide Leitungen zusammen mit dem Beutel gemeinsam steril verpackt sind.

Für den Fachmann ist dabei offensichtlich, dass das Vorkonnektieren zumindest einer Leitung mit dem Beutel unabhängig von der Anordnung der Zuleitung an einer der Leitungen von großem Vorteil ist. Dabei ist es aber offensichtlich noch vorteilhafter, wenn der erfindungsgemäße Blutschlauchsatz beide Eigenschaften aufweist. Insbesondere ist es von großem Vorteil, wenn auch die Zuleitung an einer der Leitungen entweder vorkonnektiert oder fest angeschlossen ist und gemeinsam mit diesen steril verpackt ist, da so ein weiterer Konnektiervorgang eingespart werden kann.

Vorteilhafterweise sind bei dem erfindungsgemäßen Blutschlauchsatz an den Eingängen der Kammer Ventile angeordnet. Diese sind wiederum vorteilhafterweise in Form von Klemmen ausgeführt. So kann der Beutel durch die Ventile einfach abgeschlossen werden, so dass nach dem Primen die venöse und die arterielle Leitung vom Beutel dekonnektiert und an den Patienten angeschlossen werden können ebenso läßt sich so während des Befüllens der Flüssigkeitsstrom durch die Leitungen einstellen.

Weiterhin vorteilhafterweise ist an der Zuleitung ein Ventil und/oder ein Pumpabschnitt angeordnet, das Ventil wiederum vorteilhafterweise in Form einer Klemme. Hierüber kann der Zustrom an Primingflüssigkeit gesteuert werden. Vorteilhafterweise kommt dabei ein separater Beutel mit Primingflüssigkeit oder eine Zuführung von online aus dem Dialysat gewonnener Primingflüssigkeit z.B. zum Einsatz. Der Beutel mit der Kammer mit den zwei Zuleitungen, welcher zum Zirkulieren verwendet wird und die gebrauchte Primingflüssigkeit aufnimmt, kann dann als Einkammerbeutel ausgeführt werden. Dies ermöglicht insbesondere, dass dieser vor dem Primen leer ist, so dass er einfach sterilisiert und gelagert werden kann. Zudem ist es dadurch möglich, einen flüssigkeitsfreien Blutschlauchsatz zu verwenden, welcher vorkonnektiert und gemeinsam sterilisiert und steril verpackt wird, während die Primingflüssigkeit über einen separaten Beutel oder z.B. über den Substituat-Port zugeführt wird.

Bei dem erfindungsgemäßen Primingverfahren sind für das Priming damit nur noch drei bzw. vier Konnektionsvoränge nötig. So muss lediglich der Behälter mit der Primingflüssigkeit bzw. ein Port zur Bereitstellung der Primingflüssigkeit an die Zuleitung angeschlossen werden und die filterseitigen Anschlüsse der arteriellen und der venösen Leitung mit dem Dialysator verbunden werden. Gegebenenfalls muss noch eine der Leitungen mit dem nicht vorkonnektierten Eingang des Beutels konnektiert werden. Der gesamte Befüll- und Zirkuliervorgang kann dann ohne nochmaliges Umkonnektieren von statten gehen.

Zudem benötigt das erfindungsgemäßen Verfahren mit dem erfindungsgemäßen Blutschlauchsatz keine komplizierten Verfahrensschritte, bei welchen Ventile geöffnet oder geschlossen werden müssten, oder deren Anzahl wird zumindest reduziert. Wird parallel befüllt, muss beim gesamten Primingvorgang lediglich das Ventil in der Zulaufleitung geöffnet und geschlossen werden, während die Ventile an den Eingängen der Kammer die ganze Zeit geöffnet bleiben können. Diese müssen erst geschlossen werden, wenn die venöse und die arterielle Leitung von der Kammer dekonnektiert und an den Patienten angeschlossen werden. Ist eine Pumpe für die Zuleitung vorhanden, kann der gesamte Füll- und Zirkuliervorgang auch ganz ohne das Verstellen von Ventilen allein über die Ansteuerung der Pumpen erfolgen. Wird nicht parallel befüllt, muss auch hier nur das Ventil für die arterielle Leitung nach dem Befüllen geschlossen und vor dem Zirkulieren geöffnet werden, weitere Schritte sind aber nicht nötig.

Die vorliegende Erfindung umfaßt weiterhin ein Verfahren zum Bereitstellen eines der oben beschriebenen Blutschlauchsätze, mit den Schritten: Konnektieren des leeren Beutels mit zumindest einer Leitung sowie gemeinsames Sterilisierten von Beutel und zumindest einer Leitung. So kann in einem Arbeitsschritt der gesamte vorkonnektierte Blutschlauchsatz sterilisiert werden, wobei zudem eine Kontamination durch ein erst nachträgliches Konnektieren getrennt sterilisierter Teile vermieden wird.

Der leere Beutel und damit der leere Blutschlauchsatz der vorliegenden Erfindung ermöglichen es dabei, daß diese auf einfache und kostengünstige Weise mit sämtlichen Standard-Sterilisationsverfahren sterilisiert werden können. Die lösungsgefüllten Beutel nach dem Stand der Technik können dagegen mit gewissen Standard-Sterilisationsverfahren wie z.B. der ETO-Sterilisierung nicht sterilisiert werden, da bei gefülltem Beutel zur Sterilisierung offensichtlich keine Sterilisierungsfluide verwendet werden können.

In der vorliegenden Erfindung erfolgt das gemeinsame Sterilisieren von Beutel und zumindest einer Leitung dagegen vorteilhafterweise über ein Sterilisationsfluid. So können diese einfachen und kostengünstigen Sterilisationsverfahren mit der vorliegenden Erfindung problemlos verwendet werden, da ein leerer Beutel und ein leerer Blutschlauchsatz verwendet werden.

Vorteilhafterweise erfolgt das gemeinsame Sterilisieren von Beutel und zumindest einer Leitung dabei mit ETO (Ethylen Oxid). Dies ist ein häufig verwendetes Sterilisationsgas, mit welchem ein preiswertes Sterilisationsverfahren realisiert werden kann.

Die vorliegende Erfindung umfasst weiterhin die Verwendung eines Beutels für das Primen des Blutschlauchsets, mit einer Kammer, zwei separaten Eingängen zu der Kammer an der unteren Seite des Beutels sowie einem Aufhängelement zum Aufhängen des Beutels an einer oberen Seite des Beutels. Dabei sind die beiden Eingänge bezüglich einer vertikalen Mittellinie der Kammer auf einer Seite und das Aufhängelement auf der anderen Seite angeordnet. Hierdurch läßt sich insbesondere der erfindungsgemäße Zirkulierschritt auch mit einer nur sehr geringen Menge an Primingflüssigkeit durchführen, da durch die in einer Ecke angeordneten Eingänge sowie das in der diagonal gegenüberliegenden Ecke angeordnete Aufhängelement der Beutel schräg zum Hängen kommt und sich die Primingflüssigkeit so in der Ecke mit den Eingängen sammelt. Hierdurch ergibt sich zudem eine besonders günstige Strömung innerhalb des Beutels, welche die Luftabscheidung sowie die Ablagerung von ausgespülten Teilchen unterstützt. Dabei ist es möglich, durch entsprechendes Auffüllen des Beutels eine beliebig große Menge an Primingflüssigkeit zum zirkulieren zu verwenden, so dass diese Menge je nach Sterilisationsverfahren und/oder verwendetem Filter bzw. Füllstoffen des Filters aus der Produktion eingestellt werden kann. Eine auf dem Beutel angebrachte Skala kann hier zur einfacheren Bedienung beitragen.

Vorteilhafterweise weisen Eingänge sowie das Aufhängelement dabei einen Abstand von der vertikalen Mittellinie der Kammer auf, der größer oder gleich einem Sechstel der Breite der Kammer ist. Durch diese Anordnung, bei welcher im mittleren Drittel der Kammer weder die Eingänge noch das Aufhängelement angeordnet sind, kann erreicht werden, dass der Beutel beim Aufhängen optimal schräg hängt und sich die Flüssigkeit oberhalb der Eingänge sammelt. Hierdurch wird nur wenig physiologische Flüssigkeit benötigt, um die beiden innen liegenden Enden der Eingänge zu überdecken. Diese Überdeckung ist aber wegen der Vermeidung von Luftzirkulation notwendig.

Weiterhin vorteilhafterweise besteht der Beutel aus zwei miteinander verschweißten Lagen Plastikfolie, wobei das Aufhängelement in einer oberen Ecke des Beutels angeordnet ist und durch eine schräg verlaufende Schweißnaht gebildet wird. So kann auf einfache Art und Weise eine stabile und sichere Aufhängung des Beutels gesichert werden. Das Aufhängelement kann dabei z. B. eine Lochung in der den Beutel bildenden Folie sein bzw. eine entsprechende Perforation oder runde Schweißnaht sein, welche noch durchstochen werden muss.

Vorteilhafterweise ist bei dem erfindungsgemäßen Beutel nur eine Kammer vorgesehen. Dieser Einkammerbeutel hat den Vorteil, dass sich bezüglich der Anschlüsse keine Verwechslungsgefahr ergibt. Die zum Primen benötigte physiologische Flüssigkeit kommt dann aus einem anderen Beutel. So kann der erfindungsgemäße Beutel auch besser sterilisiert und gelagert werden.

Weiterhin vorteilhafterweise ragen bei dem erfindungsgemäßen Beutel die Eingänge schlauchartig in die Kammer hinein. Dies unterstützt die Ablagerung von ausgespülten Teilchen und Fremdkörpern im Beutel sowie die Luftabscheidung.

Vorteilhafterweise sind bei dem erfindungsgemäßen Beutel an jedem der zwei Eingänge eine Klemme zum Verschließen des jeweiligen Eingangs angeordnet. Über diese Klemme kann so vor dem Dekonnektieren der venösen und der arteriellen Leitung der Beutel verschlossen werden, so dass keine Primingflüssigkeit ausläuft. Die Verwendung von Klemmen ergibt so eine einfache und übersichtliche Handhabung des Beutels.

Weiterhin vorteilhafterweise weisen die Eingänge jeweils einen Anschluß zum Anschließen an einen Blutschlauchsatz auf. Über diese Anschlüsse können die beiden separaten Eingänge einfach und sicher an die patientenseitigen Anschlüsse der venösen und der arteriellen Leitung angeschlossen werden.

Weiterhin vorteilhafterweise ist auf dem erfindungsgemäßen Beutel eine Skala angebracht. Diese kann z. B. auf den Beutel aufgedruckt sein. Vorteilhafterweise zeigt diese Skala den Flüssigkeitsstand im Beutel an und hilft so dem Personal, die einzelnen Primingschritte zu überwachen.

Weiterhin vorteilhafterweise ist der erfindungsgemäße Beutel vor dem Primen leer und steril verpackt. So ergeben sich einerseits Gewichtsvorteile bei der Lagerung und der Auslieferung sowie Vorteile bei der sterilen Verwendung des erfindungsgemäßen Beutels.

Wie bereits im Bezug auf den Blutschlauchsatz ausgeführt, ermöglicht der leere Beutel der vorliegenden Erfindung eine einfache und kostengünstige Sterilisation mit sämtlichen Standard-Sterilisationsverfahren. Die lösungsgefüllten Beutel nach dem Stand der Technik können dagegen mit gewissen Standard-Sterilisationsverfahren wie z.B. der ETO-Sterilisierung nicht sterilisiert werden, da bei gefülltem Beutel zur Sterilisierung offensichtlich keine Sterilisierungsfluide verwendet werden können.

Die vorliegende Erfindung umfaßt dagegen weiterhin ein Verfahren zur Bereitstellung eines der oben beschriebenen Beutel, wobei der Beutel mit einem Sterilisationsfluid, insbesondere mit ETO, sterilisiert wird. So können diese einfachen und kostengünstigen Sterilisationsverfahren mit der vorliegenden Erfindung problemlos verwendet werden, da ein leerer Beutel verwendet wird.

Für den Fachmann ist dabei offensichtlich, dass es von großem Vorteil ist, den erfindungsgemäßen Beutel für den erfindungsgemäßen Blutschlauchsatz bzw. für das erfindungsgemäße Verfahren zu verwenden. Ebenso ist es von großem Vorteil, den erfindungsgemäßen Blutschlauchsatz für das erfindungsgemäße Verfahren zu verwenden. Dabei können auch nur einzelne Aspekte des erfindungsgemäßen Beutels für den erfindungsgemäßen Blutschlauchsatz verwendet werden wie z. B. die erfindungsgemäße Skala. Ebenso verhält es sich mit den einzelnen Merkmalen des Beutels und des Blutschlauchsatzes, welche ebenso vorteilhaft bei dem erfindungsgemäßen Verfahren zum Einsatz kommen können.

Die vorliegende Erfindung wird nun anhand eines Ausführungsbeispiels und Zeichnungen näher erläutert. Dabei zeigen:
- Figur 1:: ein Prinzipschaubild eines ersten Ausführungsbeispiels des erfindungsgemäßen Verfahrens unter Verwendung des erfindungsgemäßen Blutschlauchsatzes,
- Figur 2:: ein Prinzipschaubild eines zweiten Ausführungsbeispiels des erfindungsgemäßen Verfahrens unter Verwendung des erfindungsgemäßen Blutschlauchsatzes, und
- Figur 3:: den erfindungsgemäßen Beutel.

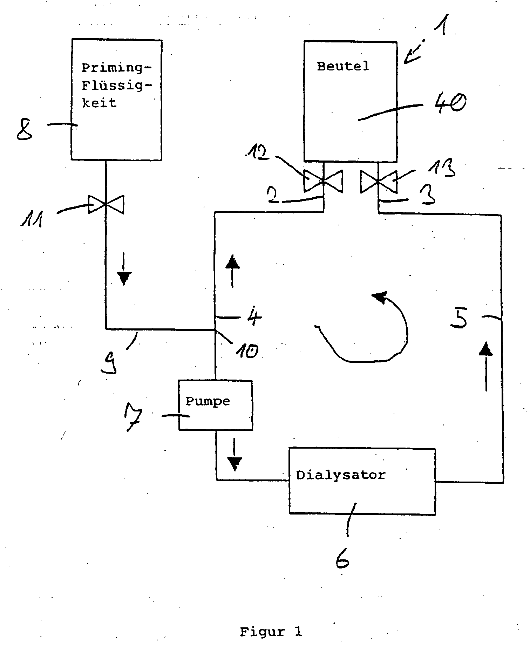

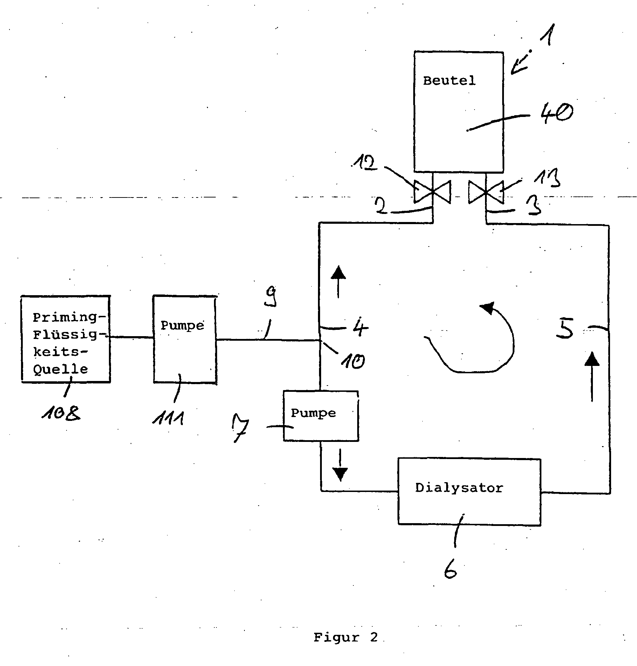

Figur 1 und Figur 2 zeigen ein Prinzipschaubild eines ersten und zweiten Ausführungsbeispiels des erfindungsgemäßen Verfahrens zum Primen eines Blutschlauchsatzes sowie den entsprechenden erfindungsgemäßen Blutschlauchsatz. Der Blutschlauchsatz besteht dabei aus einer arteriellen Leitung 4 sowie einer venösen Leitung 5, welche patientenseitig mit den Eingängen 2 und 3 des Beutels 1 verbunden sind. Der Beutel 1 ist dabei so angeordnet, dass die beiden Eingänge 2 und 3, welche separat in die von dem Beutel gebildete Kammer 40 hineinreichen, an der unteren Seite des Beutels angeordnet sind. Die Eingänge 2 und 3 lassen sich dabei über Ventile 12 und13 öffnen bzw. verschließen.

Mit der arteriellen Leitung 4 ist an einem Zuleitungspunkt 10 die Zuleitung 9 verbunden, wobei sie entweder in die Leitung integriert oder lösbar mit dieser verbunden sein kann. Es handelt sich dabei um eine T-Verbindung, über welche Primingflüssigkeit über die Zuleitung 9 in die arterielle Leitung 4 fließen kann.

Die arterielle Leitung 4 sowie die venöse Leitung 5 sind filterseitig mit der Blutseite des Dialysators 6 verbunden. Über diesen Dialysator 6 kann so das während der extrakorporalen Bluttherapie durch den Blutschlauchsatz fließende Blut gereinigt bzw. filtriert werden. Zwischen dem Zuleitungspunkt 10 der Zuleitung 9 in die arterielle Leitung 4 und dem Dialysator 6 ist dabei die Pumpe 7 angeordnet, so dass aus der Zuleitung 9 während des Primings zufließende Primingflüssigkeit einerseits direkt zum Eingang 2 des Beutels 1 fließen kann, andererseits über die Pumpe 7 zuerst zum Dialysator 6 und dann über die venöse Leitung 5 zum Eingang 3 des Beutels 1 gepumpt werden kann. Die Pumpe 7 ist dabei vorteilhafterweise eine Schlauchpumpe, welche an einem Pumpsegment in der arteriellen Leitung 4 angreift.

Erfindungsgemäß wird der Blutschlauchsatz aus Zuleitung 9, arterieller Leitung 4, Beutel 1 und venöser Leitung 5 dabei teilweise vorkonnektiert und gemeinsam sterilisiert. Dabei ist die Zuleitung bereits mit der arteriellen Leitung vorkonnektiert und entweder die arterielle oder die venöse Leitung mit dem Beutel vorkonnektiert. Der dann komplett steril verpackte vorkonnektierte sterile Blutschlauchsatz kann so direkt mit einer minimalen Anzahl von Konnektionsvorgängen verwendet werden, wodurch eine Kontamination des Blutschlauchsatzes effektiv vermieden wird.

Dabei ist in dem in Fig. 1 gezeigten ersten Ausführungsbeispiel an der Zuleitung 9 ein Ventil 11 vorgesehen, über welches die Zuleitung geöffnet und geschlossen werden kann. Darüber hinaus ist die Zuleitung mit einem Beutel 8 mit Primingfüssigkeit verbunden. Dieser ist erhöht angeordnet, so dass die Primingflüssigkeit nach dem Öffnen des Ventils 11 durch die Schwerkraft in den Blutschlauchsatz fließt.

Dagegen ist bei dem in Fig. 2 gezeigten zweiten Ausführungsbeispiel an der Zuleitung 9 eine Pumpe 111 vorgesehen, über welche Primingflüssigkeit durch die Zuleitung 9 in den Blutschlauchsatz gepumpt wird. Die Pumpe 111 kann dabei entweder an einem Pumpsegment in der Zuleitung 9 angreifen oder der Zuleitung vorgeschaltet sein. Die Zuleitung ist dabei mit einer Primingflüssigkeitsquelle 108 verbunden, welche wie im ersten Ausführungsbeispiel aus einem Beutel mit Flüssigkeit oder einem Port, z.B. einem Substituat-Port bestehen kann, welcher online aus dem Dialysat gewonnene Primingflüssigkeit zur Verfügung stellt.

Bevor mit dem Priming begonnen werden kann, muss der Beutel 8 mit der Primingflüssigkeit bzw. die Primingsflüssigkeitsquelle 108 an die Zuleitung 9 angeschlossen werden, die arterielle Leitung 4 in die Pumpe 7 eingelegt und mit dem Eingang des Dialysators 6 verbunden werden sowie die venöse Leitung 5 mit dem Ausgang des Dialysators verbunden werden. Die Leitungen müssen gegebenenfalls noch in weitere, hier nicht gezeigte Vorrichtungen wie z.B. Luftdetektoren eingelegt werden. Darüber hinaus muss entweder die arterielle oder die venöse Leitung mit dem Beutel konnektiert werden, da eine der beiden Leitungen nicht vorkonnektiert wird, um das Einlegen der Leitungen in die Dialysemaschine zu erleichtern.

Bei dem in Fig.1 gezeigten ersten Ausführungsbeispiel ist der Beutel 1 zu Beginn des Primens leer, während der Beutel 8 mit Primingflüssigkeit gefüllt ist. Zum Befüllen des Blutschlauchsatzes sind die Ventile 12 und 13 der Eingänge 2 und 3 des Beutels 1 geöffnet, so dass zuerst die Luft aus dem Blutschlauchsatz in diesen strömen kann. Hierzu wird das Ventil 11 in der Zuleitung 9 geöffnet, so dass Primingflüssigkeit aus dem Beutel 8 zum Zuleitungspunkt 10 der arteriellen Leitung 4 fließt. Von dort aus fließt die Primingflüssigkeit einerseits durch den Druck in der Zuleitung, welcher aufgrund der Schwerkraft besteht, direkt zum Eingang 2 des Beutels 1. Andererseits wird Flüssigkeit aus der Zuleitung 9 auch durch die Pumpe 7 in den Dialysator 6 und durch diesen hindurch in die venöse Leitung 5 gepumpt, von wo aus die Primingflüssigkeit über den Eingang 3 des Beutels 1 in diesen fließt. Die Primingflüssigkeit verdrängt so in den Leitungen die Luft, welche sich in diesen befand. Im übrigen sind dabei auch andere Zuleitpunkte 10 denkbar, z. B. zwischen Pumpe 7 und Dialysator 6. Die Pumpe 7 muss dann zum Befüllen der Leitung lediglich rückwärts laufen. Dabei ist insbesondere das getrennte Befüllen der Leitungen von Vorteil, bei welchem zuerst die arterielle Leitung 4 vom Zuleitpunkt 10 ab bei ausgeschalteter Pumpe 7 befüllt wird, bis die Primingflüssigkeit die gesamte Luft aus diesem Abschnitt verdrängt hat und über den Eingang 2 in den Beutel 1 fließt. Daraufhin wird das Ventil 12 am Eingang 2 geschlossen, woraufhin die Pumpe 7 Primingflüssigkeit in den Rest des Blutschlauchsatzes pumpt, bis auch dieser von Luft befreit ist und die Primingflüssigkeit über den Eingang 13 in den Beutel 1 fließt. Daraufhin steigt der Flüssigkeitsspiegel in dem Beutel 1 langsam an. Hat der Flüssigkeitsspiegel in dem Beutel eine gewisse Mindesthöhe erreicht, bei welcher die inneren Enden der Eingänge 2 und 3 des Beutels 1 von Flüssigkeit bedeckt sowie über die Flüssigkeit verbunden sind, kann das Befüllen beendet werden. Es kann aber zusätzlich noch weitere Primingflüssigkeit eingefüllt werden, um die beim nächsten Schritt zu zirkulierende Flüssigkeitsmenge zu erhöhen und den Blutschlauchsatz sowie den Dialysator besser zu spülen. Zum Zirkulieren wird dann das Ventil 12 wieder geöffnet.

Alternativ könne aber auch im ersten Ausführungsbeispiel beide Leitungen parallel befüllt werden, wobei jedoch eine genaue Einstellung der von der Pumpe 7 gepumpten Flüssigkeitsmenge nötig ist, da diese die über die Zuleitung aufgrund der Schwerkraft zufließende Flüssigkeitsmenge nicht überschreiten darf. So kann auf das Schließen und Öffnen des Ventils 12 verzichtet werden.

Ist der Befüllschritt beendet, wird das Ventil 11 in der Zuleitung 9 geschlossen. Die Pumpe 7 pumpt nun weiterhin Flüssigkeit durch den Dialysator 6 in die venöse Leitung 5 und darüber über den Eingang 3 in den Beutel 1. Die im Beutel 1 angesammelte Flüssigkeit wird dann über den Eingang 2, welcher nunmehr als Ausgang dient, angesaugt und so von der Pumpe 7 durch die arterielle Leitung 4 gesogen. Dieser Zirkulierschritt ist in Figur 1 als kreisförmiger Pfeil in der Mitte des Kreislaufes aus arterieller Leitung 4; Dialysator 6, venöser Leitung 5 sowie Beutel 1 dargestellt. Um den Filter des Dialysators 6 zu spülen, kann zudem ein leichter Überdruck an die Blutseite des Filters angelegt werden, so dass filtergängige Verunreinigungen ins Dialysat gespült werden. Die hierdurch verloren gehende Flüssigkeit kann während des Zirkulierens über die Zuleitung nachgefüllt werden.

Bei dem in Fig. 2 dargestellten zweiten Ausführungsbeispiels ist an der Zuleitung 9 eine Pumpe 111 vorgesehen, welche Primingflüssigkeit aus der Primingflüssigkeitsquelle 108 in die Zuleitung pumpt. Hierbei ist sowohl das oben beschriebene getrennte Befüllen von arterieller und venöser Leitung möglich, als auch das parallele Befüllen. Das parallele Befüllen wird hier aber dadurch erleichtert, dass die Pumpraten der Pumpen 111 und 7 genau angesteuert werden können. Offensichtlich müssen dabei die Pumpen 7 und 111 so eingestellt werden, dass die von der Pumpe 7 gepumpte Flüssigkeitsmenge kleiner ist als die von der Pumpe 111 in die Zuleitung 9 gepumpte Flüssigkeitsmenge. Ist die Pumpe 111 kann die Substituat-Pumpe eines Substituat-Ports, so kann mit dem zweiten Ausführungsbeispiel der Blutschlauchsatz parallel befüllt werden, ohne dass zusätzlicher apparativer Aufwand nötig wäre.

Soll der Patient nun an den extrakorporalen Blutkreislauf konnektiert werden, kann es von Vorteil sein, die gebrauchte Primingflüssigkeit, welche sich im Blutschlauchsatz befindet, durch frische Primingflüssigkeit aus dem Beutel 8 bzw. der Primingflüssigkeitsquelle 108 zu ersetzen. Hierzu wird vorgegangen wie beim ersten Befüllschritt, indem das Ventil 11 in der Zuleitung 9 geöffnet wird bzw. die Pumpe 111 betätigt wird. So fließt wiederum frische Primingflüssigkeit einerseits über die arterielle Leitung 4 rückwärts zum Eingang 2 des Beutels 1, während in der anderen Richtung die über die Zuleitung 9 zugeleitete Primingflüssigkeit von der Pumpe 7 über den Dialysator 6 und die venöse Leitung 5 zum Eingang 3 des Beutels gepumpt wird. Ist der gesamte extrakorporale Blutkreislauf mit frischer Primingflüssigkeit gefüllt; wird das Ventil 11 in der Zuleitung 9 wieder geschlossen bzw. die Pumpe 111 nicht weiter betätigt.

Zum Anschließen des Patienten an den extrakorporalen Blutkreislauf wird nun zunächst das Ventil 12 des Eingangs 2 geschlossen und der patientenseitige Anschluß der arteriellen Leitung 4 von dem entsprechenden Anschluß des Eingangs 2 getrennt. Der patientenseitige Anschluß der arteriellen Leitung 4 wird nun mit dem Patienten konnektiert, woraufhin die Pumpe 7 zunächst die verbleibende Primingflüssigkeit und daraufhin Blut über die arterielle Leitung 4 durch den Dialysator 6 in die venöse Leitung 5 pumpt. Das einströmende Blut des Patienten verdrängt dabei die Primingflüssigkeit im Blutschlauchsatz. Der patientenseitige Anschluß der venösen Leitung 5 kann dabei so lange mit dem Eingang 3 des Beutels 1 verbunden bleiben, bis die gesamte Primingflüssigkeit, welche noch im Blutschlauchsatz vorhanden war, in den Beutel 1 geflossen ist. So kann auch auf das Befüllen des Blutschlauchsatzes mit frischer Primingflüssigkeit ganz oder teilweise verzichtet werden, da diese ohnehin in den Beutel abfließt. Daraufhin wird das Ventil 13 des Eingangs 3 geschlossen und auch die venöse Leitung 5 von dem Eingang 3 getrennt und mit dem Patienten verbunden. Nun kann die eigentliche extrakorporale Blutbehandlung beginnen.

Alternativ kann, da durch das Nachfüllen des Blutschlauchsatzes mit frischer Primingflüssigkeit dieser ohnehin mit sauberer physiologischer Lösung gefüllt ist, das Konnektieren der venösen und der arteriellen Leitung mit dem Patienten auch gleichzeitig erfolgen, so dass dem Patienten keine Flüssigkeit entzogen wird, sondern ihm die gleiche Menge physiologischer Flüssigkeit zugeführt wird, wie ihm an Blut entzogen und in den Blutschlauchsatz gepumpt wird. Insbesondere bei Patienten mit schwachem Kreislauf kann dies vorteilhaft sein.

Das Vorhandensein der Zuleitung 9 zu dem extrakorporalen Blutkreislauf hat auch während der Behandlung den Vorteil, dass dem Patienten jederzeit Flüssigkeit z.B. als Bolusinfusion bei Kreislaufschwäche zugeführt werden kann. Zudem ist es möglich, durch Zugabe von Primingflüssigkeit z. B. im Dialysator durch Hämofiltration verlorengegangene Flüssigkeit zumindest teilweise zu ersetzen. Dies geschieht üblicherweise dadurch, dass nach dem dekonnektieren der arteriellen Leitung vom Patienten noch entsprechend viel Primingflüssigkeit aus der Zuleitung zugeführt wird.

Nach der Behandlung wird dabei zunächst der patientenseitige Anschluß der arteriellen Leitung 4 vom Patienten getrennt. Um das noch im Blutschlauchsatz verbliebene Blut wieder dem Patienten zuzuführen, kann nun wieder Primingflüssigkeit über die Zuleitung 9 in die arterielle Leitung 4 gegeben werden, welche das Blut im Blutkreislauf oberhalb des Zuleitungspunktes 10 verdrängt. So geht nur eine geringe Menge an Blut verloren, da der patientenseitige Anschluß der venösen Leitung 5 erst dann vom Patienten abgenommen wird, wenn die Primingflüssigkeit aus der Zuleitung 9 das Blut komplett aus der venösen Leitung verdrängt hat. Hierbei kann auch noch eine gewisse Menge an Primingflüssigkeit dem Patienten zugeführt werden, um eventuelle Flüssigkeitsverluste ganz oder teilweise auszugleichen.

So kommt man während des gesamten Vorgangs mit einem Minimum an Konnektiervorgängen und Umschaltvorgängen und nur einem einzigen Beutel Primingflüssigkeit bzw. einer einzigen Primingflüssigkeitsquelle aus, was einerseits die Sicherheit erhöht und andererseits Kosten spart. Auch werden Fehlbedienungen durch das Personal effektiv verhindert.

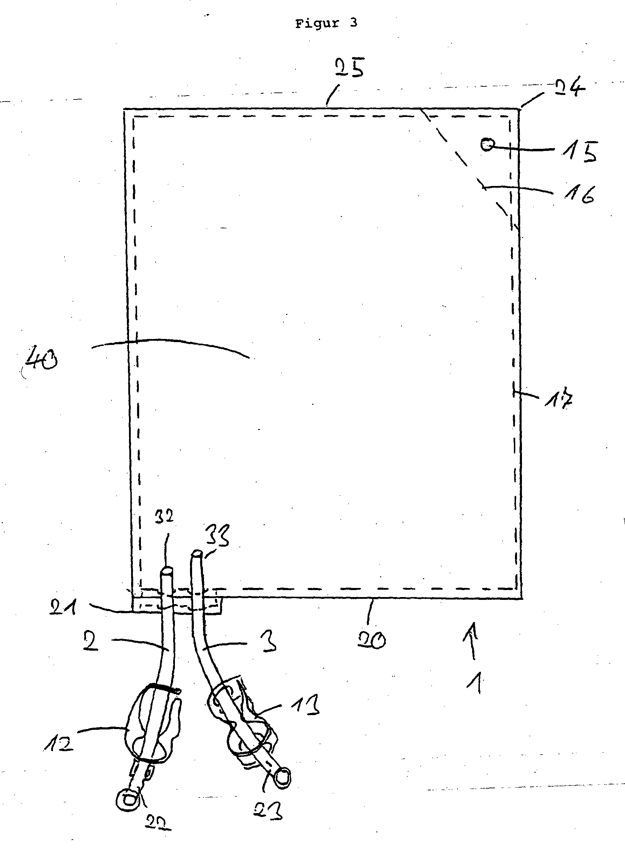

Figur 3 zeigt nun ein Ausführungsbeispiel eines erfindungsgemäßen Beutels 1, welcher vorteilhafterweise bei dem erfindungsgemäßen Verfahren bzw. in dem erfindungsgemäßen Blutschlauchsatz zum Einsatz kommt. Der Beutel 1 besteht dabei aus zwei Lagen von Plastikfolie, welche in ihren Randbereichen durch Schweißnähte 17 zusammengeschweißt sind und so eine einzige Kammer 40 bilden. In diese Kammer 40 ragen die Enden 32 und 33 der Eingänge 2 und 3 hinein. Dies hat insbesondere den Vorteil, dass sich Fremdstoffe und Partikel während des Zirkulierens im Beutel an dessen unteren Seite 20 ansammeln und nicht mehr in den Blutschlauchsatz gespült werden.

Die beiden Eingänge 2 und 3 sind dabei in dem Ausführungsbeispiel links von einer vertikalen Mittellinie des Beutels angeordnet. Im Gegenzug dazu ist das Aufhängelement 15, welches hier durch ein einfaches Loch gebildet ist, rechts von dieser vertikalen Mittellinie in der rechten oberen Ecke an der oberen Seite 25 des Beutels angeordnet. Wird nun der Beutel 1 mittels des Aufhängelements 15 an einen Haken gehängt, fließt die Flüssigkeit im Beutel automatisch in die linke untere Ecke, in welcher die Enden 32 und 33 der Eingänge 2 und 3 angeordnet sind. So kann auch mit einem Minimum an Flüssigkeit in dem Beutel eine Abdeckung der beiden Eingänge 2 und 3 mit Flüssigkeit erreicht werden, welche für das Zirkulieren der Flüssigkeit über den Beutel unabdingbar ist. Zudem ergibt sich durch diese schräge Anordnung eine günstige Flüssigkeitsströmung in dem Beutel.

Das Aufhängelement 15 ist dabei in der rechten oberen Ecke 24 des Beutels angeordnet und durch eine schräge Schweißnaht 16 von der Kammer 40 des Beutels getrennt. Die beiden Eingänge 2 und 3 sind schlauchförmig ausgeführt und weisen an ihren vom Beutel abgewandeten Enden Anschlüsse 22 und 23 zum Anschluß an die entsprechenden Gegenstücke der venösen und der arteriellen Leitung auf. Zudem sind an den Eingängen 2 und 3 jeweils Klemmen 12 und 13 angebracht, über welche sich der Flüssigkeitsstrom durch die Eingänge 2 und 3 regulieren läßt.

Der zuvor beschriebene Beutel für das Primen eines Blutschlauchsets umfaßt demnach eine Kammer 40, zwei separaten Eingänge 2,3 zu der Kammer 40 an einer unteren Seite 20 des Beutels sowie ein Aufhängelement 15 zum Aufhängen des Beutels an einer oberen Seite 25 des Beutels, wobei die Eingänge 2,3 bezüglich einer vertikalen Mittellinie der Kammer 40 auf einer Seite und das Aufhängelement 15 auf der anderen Seite angeordnet sind.

Die Eingänge 2,3 des Beutels sowie das Aufhängelement 15 weisen einen Abstand von der vertikalen Mittellinie der Kammer 40 auf, der größer oder gleich einem Sechstel der Breite der Kammer ist.

Der Beutel kann aus zwei miteinander verschweißten Lagen Plastikfolie bestehen und das Aufhängelement 15 kann in einer oberen Ecke 24 des Beutels angeordnet sein, die durch eine schräg verlaufende Schweißnaht 16 gebildet wird.

Der Beutel kann nur eine Kammer 40 aufweisen. Die Eingänge 2,3 können schlauchartig in die Kammer hineinragen.

Es kann jeweils eine Klemme 12, 13 zum Verschließen der Eingänge 2,3 vorgesehen sein.

Die Eingänge 2,3 des Beutels können jeweils einen Anschluß 22,23 zum Anschließen an einen Blutschlauchsatz aufweisen.

Auf dem Beutel kann eine Skala angebracht sein. Er kann leer und steril verpackt sein. Der Beutel kann mit einem Sterilisationsfluid, insbesondere mit ETO, sterilisiert werden.

## Patentansprüche

1. Verfahren zum Primen eines Blutschlauchsatzes aus einer venösen Leitung (5) und einer arteriellen Leitung (4), deren patientenseitige Anschlüsse mit zwei separaten Eingängen (2,3) einer Kammer (40) insbesondere eines Beutels (1) in Verbindung stehen und deren filterseitige Anschlüsse mit einem Dialysator (6) in Verbindung stehen, mit den Schritten,
- Befüllen der venösen Leitung (5) und der arteriellen Leitung (4) über eine Zuleitung (9), wobei Primingflüssigkeit über die Leitungen (4,5) durch die Eingänge (2,3) in die Kammer (40) fließt, wobei die Kammer (40) zu Beginn des Primings leer ist,
- Zirkulieren der Primingflüssigkeit in dem Kreislauf aus den Leitungen (4,5), dem Dialysator (6) und der Kammer (40) über eine Pumpe (7), so dass einer der Eingänge (2,3) der Kammer (40) als Eingang (3) und der andere als Ausgang (2) fungiert.

2. Verfahren nach Anspruch 1, wobei nach dem Zirkulieren die gebrauchte Primingflüssigkeit im Blutschlauchsatz durch Nachfüllen frischer Primingflüssigkeit aus der Zuleitung (9) in die venöse Leitung (5) und in die arterielle Leitung (4) ersetzt wird, wobei die gebrauchte Primingflüssigkeit durch die Eingänge (2,3) in die Kammer (40) fließt.

3. Verfahren nach Anspruch 1 oder 2, wobei beim Befüllen oder Nachfüllen die Primingflüssigkeit über die Zuleitung (9) stromabwärts einer Pumpe (7) in einer der Leitungen (4,5) zugegeben wird.

4. Verfahren nach einem der vorangegangenen Ansprüche, wobei die beiden Eingänge (2,3) der Kammer (40) jeweils über Ventile (12,13) geöffnet und geschlossen werden können.

5. Verfahren nach einem der vorangegangenen Ansprüche, wobei zunächst bei ausgeschalteter Pumpe (7) der Abschnitt des Blutschlauchsatzes ohne Pumpe, insbesondere die arterielle Leitung (4), befüllt wird, und daraufhin der Abschnitt mit Pumpe, insbesondere die venöse Leitung (5), durch Betreiben der Pumpe befüllt wird, wobei vorteilhaft ein an dem Eingang (2), welcher mit dem patientenseitigen Anschluß der arteriellen Leitung (4) verbunden ist, angeordnetes Ventil geschlossen wird, um die venöse Leitung (5) über die Pumpe (7) zu befüllen.

6. Verfahren nach einem der Ansprüche 1 bis 4, wobei sowohl die arterielle Leitung (4) als auch die venöse Leitung (5) parallel befüllt werden, indem Primingflüssigkeit aus der Zuleitung (9) gleichzeitig in der Abschnitt ohne Pumpe läuft und von der Pumpe in den Abschnitt mit Pumpe gepumpt wird.

7. Verfahren nach einem der vorangegangenen Ansprüche, wobei die Zuleitung (9) über ein Ventil (11) geöffnet und geschlossen werden kann.

8. Verfahren nach einem der vorangegangenen Ansprüche, wobei die Primingflüssigkeit über eine Pumpe (111) durch die Zuleitung gepumpt wird.

9. Verfahren nach einem der vorangegangenen Ansprüche, wobei die Zuleitung (9) mit einer zweiten Kammer (8) insbesondere eines zweiten Beutels mit Primingflüssigkeit in Verbindung steht.

10. Verfahren nach einem der Ansprüche 1 bis 8, wobei die in die Zuleitung (9) geleitete Primingflüssigkeit on-line aus dem Dialysat gewonnen wird.

11. Verfahren nach einem der Ansprüche 1 bis 8, wobei die Zuleitung mit einem Substituat-Port in Verbindung steht.

12. Verfahren nach einem der vorangegangenen Ansprüche, wobei ein Beutel (1) und ein Blutschlauchsatz zum Einsatz kommen, die zumindest teilweise vorkonnektiert und in ihrer Gesamtheit sterilisiert sind.

13. Verfahren nach einem der vorangegangenen Ansprüche, wobei ein Beutel (1) mit nur einer Kammer verwendet wird.

14. Blutschlauchsatz mit einem Beutel (1) mit einer Kammer (40) mit zwei separaten Eingängen (2,3) sowie mit einer venösen Leitung (5) und einer arteriellen Leitung (4), welche mit den zwei separaten Eingängen (2,3) lösbar verbindbar sind, wobei an einer der Leitungen eine Zuleitung (9) angeordnet ist,
**dadurch gekennzeichnet,**
**dass** zumindest eine der Leitungen (4,5) mit dem leeren Beutel (1) vorkonnektiert und mit diesem gemeinsam steril verpackt ist.

15. Blutschlauchsatz nach Anspruch 14, wobei an der Zuleitung (9) ein Ventil (11) und/oder ein Pumpabschnitt (111) angeordnet ist.

16. Blutschlauchsatz nach Anspruch 14 oder 15, wobei an den Eingängen (2,3) Ventile (12,13) angeordnet sind.

17. Verfahren zum Bereitstellen eines Blutschlauchsatzes nach einem der Ansprüche 14 bis 16, mit den Schritten:
- Konnektieren des leeren Beutels mit zumindest einer venösen oder arteriellen Leitung (4,5), wobei an einer der Leitungen eine Zuleitung angeordnet ist
- gemeinsames Sterilisieren und steriles Verpacken von Beutel und zumindest einer der Leitungen (4,5).

18. Verfahren gemäß Anspruch 17, wobei das gemeinsame Sterilisieren von Beutel und zumindest einer Leitung (4,5) über ein Sterilisationsfluid, vorzugsweise ETO, erfolgt.

## Claims

1. A method for priming a blood tubing set comprising a venous line (5) and an arterial line (4) whose connections at the patient side are in communication with two separate inlets (2, 3) of a chamber (40) of in particular a bag (1) and whose connections at the filter side are in communication with a dialyzer (6) comprising the steps,
- filling of the venous line (5) and the arterial line (4) via a feed line (9), with priming liquid flowing through the inlets (2, 3) into the chamber (40) via the lines (4, 5), wherein the chamber (40) is empty at the start of the priming,
- circulating of the priming liquid in the circuit of the lines (4, 5), the dialyzer (6) and the chamber (40) via a pump (7) so that one of the inlets (2, 3) of the chamber (40) acts as an inlet (3) and the other as an outlet (2).

2. A method in accordance with claim 1, wherein the consumed priming liquid in the blood tubing set is replaced after circulation by refilling fresh priming liquid from the feed line (9) into the venous line (5) and into the arterial line (4), with the consumed priming liquid flowing through the inlets (2, 3) into the chamber (40).

3. A method in accordance with either of claims 1 or 2, wherein, on filling or refilling, the priming liquid is added downstream of a pump (7) in one of the lines (4, 5) via the feed line (9).

4. A method in accordance with one of the preceding claims, wherein the two inlets (2, 3) of the chamber (40) can each be opened and closed via valves (12, 13).

5. A method in accordance with one of the preceding claims, wherein the portion of the blood tubing set without pump, in particular the arterial line (4), is first filled with the pump (7) switched off and the portion with pump, in particular the venous line (5), is thereupon filled by operating the pump, wherein a valve which is arranged at the inlet (2) which is connected to the connection of the arterial line (4) at the patient side is advantageously closed to fill the venous line (5) via the pump (7).

6. A method in accordance with one of the claims 1 to 4, wherein both the arterial line (4) and the venous line (5) are filled in parallel in that priming liquid runs out of the feed line (9) in the portion without pump and is simultaneously pumped by the pump into the portion with pump.

7. A method in accordance with one of the preceding claims, wherein the feed line (9) can be opened or closed via a valve (11).

8. A method in accordance with one of the preceding claims, wherein the priming liquid is pumped through the feed line via a pump (111).

9. A method in accordance with one of the preceding claims, wherein the feed line (9) is in communication with a second chamber (8) of in particular a second bag with priming liquid.

10. A method in accordance with any one of the claims 1 to 8, wherein the priming liquid guided into the feed line (9) is gained online from the dialysate.

11. A method in accordance with any one of the claims 1 to 8, wherein the feed line is in communication with a substituate port.

12. A method in accordance with one of the preceding claims, wherein a bag (1) and a blood tubing set are used which are at least partly pre-connected and are sterilized in their totality.

13. A method in accordance with one of the preceding claims, wherein a bag (1) with only one chamber is used.

14. A blood tubing set having a bag (1) with a chamber (40) having two separate inlets (2, 3) and having a venous line (5) and an arterial line (4) which are detachably connectable to the two separate inlets (2, 3), wherein a feed line (9) is arranged at one of the lines,
**characterized in that**
at least one of the lines (4, 5) is pre-connected to the empty bag (1) and is packed together with it in a sterile manner.

15. A blood tubing set in accordance claim 14, wherein a valve (11) and/or a pump portion (111) is/are arranged at the feed line (9).

16. A blood tubing set in accordance with claim 14 or 15, wherein valves (12, 13) are arranged at the inlets (2, 3).

17. A method for the provision of a blood tubing set in accordance with any of the claims 14 to 16 comprising the steps:
- connecting the empty bag to at least one venous or arterial line (4, 5), wherein a feed line is arranged at one of the lines,
- common sterilization and sterile packing of the bag and at least one of the lines (4, 5).

18. A method in accordance with claim 17, wherein the common sterilization of the bag and at least one line (4, 5) takes place via a sterilization fluid, preferably ETO.

## Revendications

1. Procédé d'amorçage d'un ensemble de tubes sanguins constitué d'une ligne veineuse (5) et d'une ligne artérielle (4), dont les connexions côté patient sont en liaison avec deux entrées distinctes (2, 3) d'une chambre (40) en particulier d'une poche (1) et dont les connexions côté filtre sont en liaison avec un dialyseur, comprenant les étapes consistant à :
- remplir la ligne veineuse (5) et la ligne artérielle (4) via une conduite d'amenée (9), du liquide d'amorçage s'écoulant via les lignes (4, 5) dans la chambre (40) à travers les entrées (2, 3), la chambre (40) étant vide au début de l'amorçage,
- faire circuler le liquide d'amorçage dans le circuit constitué des lignes (4, 5), du dialyseur (6) et de la chambre (40) au moyen d'une pompe (7), de sorte que l'une des entrées (2, 3) de la chambre (40) serve d'entrée (3) et que l'autre serve de sortie (2).

2. Procédé selon la revendication 1, dans lequel après la circulation, le liquide d'amorçage usé dans l'ensemble de tubes sanguins est remplacé en ajoutant du liquide d'amorçage frais venant de la conduite d'amenée (9) dans la ligne veineuse (5) et la ligne artérielle (4), le liquide d'amorçage usé s'écoulant dans la chambre (40) à travers les entrées (2, 3).

3. Procédé selon la revendication 1 ou 2, dans lequel lors du remplissage ou de l'ajout, le liquide d'amorçage est ajouté via la conduite d'amenée (9) en aval de la pompe (7) dans l'une des lignes (4, 5).

4. Procédé selon l'une des revendications précédentes, dans lequel les deux entrées (2, 3) de la chambre (4) peuvent être respectivement ouvertes et fermées par des soupapes (12, 13).

5. Procédé selon l'une des revendications précédentes, dans lequel d'abord la section sans pompe de l'ensemble de tubes sanguins, en particulier la ligne artérielle (4), est remplie avec la pompe arrêtée (7), puis la section avec pompe, en particulier la ligne veineuse (5), est remplie par l'opération de la pompe, une soupape agencée à l'entrée (2) qui est reliée à la connexion côté patient de la ligne artérielle (4) étant avantageusement fermée, pour remplir la ligne veineuse (5) au moyen de la pompe (7).

6. Procédé selon l'une des revendications 1 à 4, dans lequel la ligne artérielle (4) ainsi que la ligne veineuse (5) sont remplies parallèlement, en ce que du liquide d'amorçage venant de la conduite d'amenée (9) s'écoule dans la section sans pompe et est pompé en même temps dans la section avec pompe par la pompe.

7. Procédé selon l'une des revendications précédentes, dans lequel la conduite d'amenée (9) peut être ouverte et fermée par une soupape (11).

8. Procédé selon l'une des revendications précédentes, dans lequel le liquide d'amorçage est pompé à travers la conduite d'amenée au moyen d'une pompe (111).

9. Procédé selon l'une des revendications précédentes, dans lequel la conduite d'amenée (9) est en liaison avec une seconde chambre (8) en particulier d'une seconde poche avec du liquide d'amorçage.

10. Procédé selon l'une des revendications 1 à 8, dans lequel le liquide d'amorçage alimenté dans la conduite d'amenée (9) est obtenu en ligne à partir du dialysat.

11. Procédé selon l'une des revendications 1 à 8, dans lequel la conduite d'amenée est en liaison avec un port pour solution de substitution.

12. Procédé selon l'une des revendications précédentes, dans lequel on utilise une poche (1) et un ensemble de tubes sanguins qui sont au moins en partie préconnectés et dans leur ensemble stérilisés.

13. Procédé selon l'une des revendications précédentes, dans lequel une poche (1) à chambre unique est utilisée.

14. Ensemble de tubes sanguins comprenant une poche (1) avec une chambre (40) comportant deux entrées distinctes (2, 3), ainsi qu'une ligne veineuse (5) et une ligne artérielle (4) qui peuvent être reliées de manière détachable aux deux entrées distinctes (2, 3), dans lequel une conduite d'amenée (9) est agencée sur l'une des lignes, **caractérisé en ce qu'**au moins l'une des lignes (4, 5) est préconnectée avec la poche (1) vide et est emballée conjointement avec celle-ci de manière stérile.

15. Ensemble de tubes sanguins selon la revendication 14, dans lequel une soupape (11) et/ou une section de pompe (111) est agencée sur la conduite d'amenée (9).

16. Ensemble de tubes sanguins selon la revendication 14 ou 15, dans lequel des soupapes (12, 13) sont agencées sur les entrées (2, 3).

17. Procédé permettant d'obtenir un ensemble de tubes sanguins selon l'une des revendications 14 à 16, comprenant les étapes consistant à :
- connecter la poche vide avec au moins une ligne veineuse ou artérielle (4, 5), une conduite d'amenée étant agencée sur l'une des lignes
- conjointement stériliser et emballer de manière stérile la poche et au moins l'une des lignes (4, 5).

18. Procédé selon la revendication 17, dans lequel la stérilisation conjointe de la poche et d'au moins une ligne (4, 5) est effectuée au moyen d'un fluide de stérilisation, de préférence de l'EtO.
